# EUROPEAN PATENT SPECIFICATION

(11) **EP 3 112 864 B1**
(45) Date of publication and mention of the grant of the patent: **24.08.2022**
(21) Application number: 15755678.8
(22) Date of filing: 27.02.2015
(51) Int. Cl.: G01N 33/493, G01N 33/52, G01N 33/68, C12Q 1/04, G01N 21/49, G01N 21/64

(54) **URINE SAMPLE ANALYSIS METHOD AND USE OF A REAGENT KIT FOR URINE SAMPLE ANALYSIS**
VERFAHREN ZUR ANALYSE VON URINPROBEN UND VEWENDUNG EINES KITS ZUR ANALYSE VON URINPROBEN
METHODE D'ANALYSE D'ECHANTILLON D'URINE ET UTILISATION D'UNE TROUSSE DE RÉACTIFS POUR L'ANALYSE D'ECHANTILLON D'URINE

(30) Priority: 28.02.2014 JP 2014039283
(43) Date of publication of application: 04.01.2017
(73) Proprietor: Sysmex Corporation, Kobe-shi, Hyogo 651-0073 (JP)
(72) Inventor: KAWANO, Masanori, Kobe-shi Hyogo 651-0073 (JP)
(74) Representative: Hoffmann Eitle
(86) International application number: PCT/JP2015/055900
(87) International publication number: WO 2015/129870

(56) References cited:
- EP-A1- 1 865 318
- WO-A2-2006/135854
- GB-A- 2 112 285
- JP-A- H0 815 256
- JP-A- H0 843 385
- JP-A- H08 240 520
- JP-A- H09 329 596
- JP-A- 2000 221 190
- JP-A- 2001 255 260
- JP-A- 2006 149 384
- JP-A- 2007 010 685
- JP-A- 2007 255 954
- JP-A- 2010 002 428
- JP-A- 2012 132 850

## Description

### TECHNICAL FIELD

The present invention relates to a method for analyzing urine sample and the use of a reagent kit for analysis of urine sample, which are for detecting at least sperms and yeast-like fungi as urinary particles.

### BACKGROUND ART

In diseases such as infectious diseases, inflammatory lesions, degenerative lesions, calculus diseases, tumors and the like in nephric and urinary systems, a variety of particles appear in the urine depending on the individual diseases. The particles may include erythrocytes, casts, leukocytes, epithelial cells, yeast like fungi, spermatozoa and the like. Analysis of these components in the urine is important for estimating the disease and the abnormal site in nephric and urinary systems.

For example, as a reagent for detecting urinary erythrocytes, sperms and yeast-like fungi, the reagent described in JP 2007 255 954 is known. JP 2007 255 954 discloses preparing a measurement sample by treating a urine sample using a reagent containing a substance that damages yeast-like fungi without damaging erythrocytes, and a dye for discriminably staining erythrocytes, yeast-like fungi and sperms, measuring the measurement sample with a flow cytometer, and analyzing erythrocytes, yeast-like fungi and sperms discriminably on the basis of the obtained optical information. In general, an antiseptic agent is added to the reagent for analyzing urine sample in order to store the reagent stably.

Further, JP H08 240520 A describes a distinguishing apparatus for red blood corpuscle in urine for accurately and simply distinguishing an origin of red blood corpuscles in urine only by processing light signals of particles obtained from a flow sight meter. JP 2012 132850 A relates to a urine analyzer, urine specimen information processor, and method for processing urine specimen information for smoothly comparing and evaluating evaluation results. JP H09 329596 A describes a method and reagent for analysis of component having shape in urine by mixing a urine sample, a dyeing liquid which contains a fluorescent dye, a buffer agent, and an osmotic-pressure compensating agent, and a cell-membrane injuring agent. JP 2007 010685 A relates to a system and method for analyzing material component, which automate the whole or a portion of a work associated with a material component analysis of a liquid to be tested, especially, urine (urinary sediment analysis or the like) and which do not use any flow cell, having the risk of carry-over or contamination due to staining liquid. JP 2006 149384 A describes a method for producing a processed food so designed as to inhibit the breeding of triggering bacteria involved in food putrefaction, exerting antibacterial power against heat-resistant bacteria that proliferate during food storage, inhibit the growth of heat-resistant bacteria after heat sterilization, and affect neither flavor nor taste of the final food. WO 2006/135854 A2 relates to antiseptic compositions that contain DMSO or DMA, an alcohol (e.g., isopropanol), and/or an additional antiseptic agent such as iodine, wherein the antiseptic compositions may be used for skin disinfection and/or skin antisepsis (e.g., as applied via a swab). GB 2 112 285 A describes a composition for intimate feminine hygiene comprising: a) an agent which exerts a specific antiseptic action on pathogenic germs, but not on the bacillus, acidophilus such as lactic acid, acetic acid or mevalonic acid, preferably buffered, and b) a nutrient for said bacillus acidophilus, wherein the composition is said to have a beneficial physiological local effect and may be used regularly for long periods of time without the side effects normally associated with the frequent use of douches based on simple antiseptics. JP 2010 002 428 A relates to a reagent for analyzing erythrocyte in urine for efficiently and accurately determining erythrocytes even in a specimen in which a yeast-like fungus has appeared. EP 1 865 318 A1 describes a reagent for measuring basophils and/or nucleated red blood cells in a sample, which comprises at least one fluorescent dye, and also describes a kit and method for measuring basophils and/or nucleated red blood cells.

### SUMMARY OF THE INVENTION

### PROBLEMS TO BE SOLVED BY THE INVENTION

An object of the present invention is to provide a method for analyzing urine sample that can use a reagent containing an antiseptic agent and allows accurate detection of sperms and yeast-like fungi by differentiating therebetween. Further, an object of the present invention is to provide a reagent kit for analysis of urine sample that is suitably used for the above method.

### SOLUTION TO THE PROBLEMS

As a result of diligent efforts, the present inventor has found acetic acid and a salt thereof as an antiseptic agent that does not influence on discrimination between sperms and yeast-like fungi, thereby achieving the present invention.

Therefore, the present invention provides a method for analyzing urine sample comprising the steps of: preparing a measurement sample by mixing a urine sample, a first reagent containing a fluorescent dye capable of staining nucleic acid, and a second reagent containing acetic acid and/or a salt thereof as an antiseptic agent, obtaining optical information by irradiating urinary particles contained in the measurement sample obtained in the preparation step with light, and detecting at least sperms and yeast-like fungi as urinary particles on the basis of the optical information obtained in the obtention step.

Also the present invention provides the use of a reagent kit for analysis of urine sample for detecting sperms and yeast-like fungi in the urine sample by differentiating therebetween, wherein the reagent kit comprises a first reagent containing a fluorescent dye capable of staining nucleic acid, and a second reagent containing acetic acid and/or a salt thereof as an antiseptic agent and a surfactant for lysing erythrocytes and damaging urinary particles having nucleic acid so as to be permeable to the fluorescent dye.

### EFFECTS OF THE INVENTION

The present invention makes it possible to keep the storage stability of the reagent by the antiseptic agent and to accurately detect sperms and yeast-like fungi in the urine sample by differentiating therebetween.

### BRIEF DESCRIPTION OF THE DRAWINGS

Fig. 1A shows a histogram of the fluorescence intensity obtained by measuring a measurement sample containing sperms or yeast-like fungi prepared by using a diluting reagent to which a pyridine-based antiseptic agent is added.
Fig. 1B shows a histogram of the fluorescence intensity obtained by measuring a measurement sample containing sperms or yeast-like fungi prepared by using a diluting reagent to which acetic acid is added as an antiseptic agent.
Fig. 1C shows a histogram of the fluorescence intensity obtained by measuring a measurement sample containing sperms or yeast-like fungi prepared by using a diluting reagent to which a triazine-based antiseptic agent is added.
Fig. 1D shows a histogram of the fluorescence intensity obtained by measuring a measurement sample containing sperms or yeast-like fungi prepared by using a diluting reagent to which an isothiazoline-based antiseptic agent is added.
Fig. 2 shows a scattergram obtained by measuring a measurement sample containing sperms and yeast-like fungi prepared by using a diluting reagent to which acetic acid is added as an antiseptic agent.
Fig. 3 shows a scattergram obtained by measuring a measurement sample containing leukocytes prepared by using a diluting reagent to which acetic acid is added as an antiseptic agent.
Fig. 4 shows a scattergram obtained by measuring a measurement sample containing epithelial cells prepared by using a diluting reagent to which acetic acid is added as an antiseptic agent.
Fig. 5 shows a scattergram obtained by measuring a measurement sample containing atypical cells prepared by using a diluting reagent to which acetic acid is added as an antiseptic agent.
Fig. 6 shows a scattergram obtained by measuring a measurement sample containing Trichomonas prepared by using a diluting reagent to which acetic acid is added as an antiseptic agent.
Fig. 7 shows a scattergram obtained by measuring a measurement sample containing bacteria prepared by using a diluting reagent to which acetic acid is added as an antiseptic agent.
Fig. 8 is a schematic diagram of scattergram showing distributions of sperms, Trichomonas and yeast-like fungi.
Fig. 9 is a schematic diagram of scattergram showing distributions of leukocytes, atypical cells and epithelial cells.
Fig. 10 is a schematic diagram of scattergram showing a distribution of bacteria.
Fig. 11 shows one example of a reagent kit for analysis of urine sample.

### EMBODIMENTS OF THE INVENTION

### [Method for analyzing urine sample]

A method for analyzing urine sample (hereinafter, also simply referred to as "method") of the present embodiment is intended to analyze urinary particles having nucleic acid, and comprises a step of detecting at least sperms and yeast-like fungi as urinary particles. at least Examples of yeast-like fungi include fungi such as Candida. Examples of urinary particles having nucleic acid include leukocytes, epithelial cells, atypical cells, bacteria, fungi, Trichomonas and the like, besides sperms and yeast-like fungi.

In the method of the present embodiment, first, a step of preparing a measurement sample by mixing a urine sample, a first reagent containing a fluorescent dye capable of staining nucleic acid, and a second reagent containing acetic acid and/or a salt thereof as an antiseptic agent is conducted.

In the present embodiment, the urine sample is not particularly limited as far as it is a liquid sample containing the urinary particles, and is preferably the urine collected from a subject. In the case of using the urine collected from the subject as a sample, it is desired to use the urine sample for the method of the present embodiment within 24 hours, particularly within 3 to 12 hours after collection because the urinary particles may be deteriorated with time.

The first reagent used in the method of the present embodiment is a reagent for staining urinary particles including at least sperms and yeast-like fungi, and the second reagent is a reagent for diluting the urine sample. In the present embodiment, the urine sample, the first reagent, and the second reagent may be mixed in any order without limitation, and these may be mixed at the same time. In the case where the second reagent contains a later-described surfactant and/or chelating agent, it is preferable that the urine sample and the second reagent are mixed previously, and then the first reagent is further mixed therein. Alternatively, the first reagent and the second reagent may be mixed previously, and then the urine sample may be further mixed therein.

In the present embodiment, the mixing ratio of the urine sample, the first reagent, and the second reagent is not particularly limited, and may be appropriately determined depending on the component concentrations contained in the respective reagents. For example, the mixing ratio between the urine sample and the first reagent can be determined within the range of 1 : 0.01 to 1 by volume ratio. The mixing ratio between the urine sample and the second reagent can be determined within the range of 1 : 0.5 to 10 by volume ratio. The amount of the urine sample can be appropriately determined depending on the first reagent and the second reagent. The amount of the urine sample is preferably less than or equal to 1000 µL so that the measurement time is not too long. A sufficient amount of the urine sample used for the measurement is about 10 to 1000 µL.

The temperature condition in the preparation step is 10 to 60°C, preferably 37 to 44°C. Each reagent may be previously heated to the above temperature. After mixing the urine sample, the first reagent and/or the second reagent, the mixture may be incubated for 1 to 5 minutes, preferably 3 to 60 seconds.

Hereinafter, the first reagent and the second reagent for use in the preparation step of the method of the present embodiment will be described.

The first reagent contains a fluorescent dye capable of staining nucleic acid. In the present embodiment, the fluorescent dye is not particularly limited as far as it is capable of staining nucleic acid, and can be selected from the fluorescent dyes that are known in the art depending on the wavelength of the excitation light. Examples of such a fluorescent dye include an intercalator capable of specifically staining nucleic acid, and a dye that binds with minor grooves of DNA. Examples of the intercalator include cyanine-based dyes, acridine-based dyes, and phenanthridium dyes.

More specific examples of the intercalator include SYBR Green I, thiazole orange, acridine orange, propidium iodide, ethidium bromide, ethidium-acridine heterodimer, ethidium diazide, ethidium homodimer-1, ethidium homodimer-2, ethidium monoazide, trimethylene bis[[3-[[4-[[(3-methylbenzothiazole-3-ium)-2-yl]methylene]-1,4-dihydroquin oline]-1-yl]propyl]dimethy]aminium] tetraiodide (TOTO-1), 4-[(3-methylbenzothiazole-2(3H)-ylidene)methyl]-1-[3-(trimethylaminio)pro pyl]quinolinium diiodide (TO-PRO-1), N,N,N',N'-tetramethyl-N,N'-bis[3-[4-[3-[(3-methylbenzothiazole-3-ium)-2-yl] -2-propenylidene]-1,4-dihydroquinoline-1-yl]propyl]-1,3-propanediaminiu m tetraiodide (TOTO-3), and 2-[3-[[1-[3-(trimethylaminio)propyl]-1,4-dihydroquinoline]-4-ylidene]-1-pro penyl]-3-methylbenzothiazole-3-ium diiodide (TO-PRO-3). Alternatively, a fluorescent dye represented by any one of the following formulas (1) to (3) may be used. 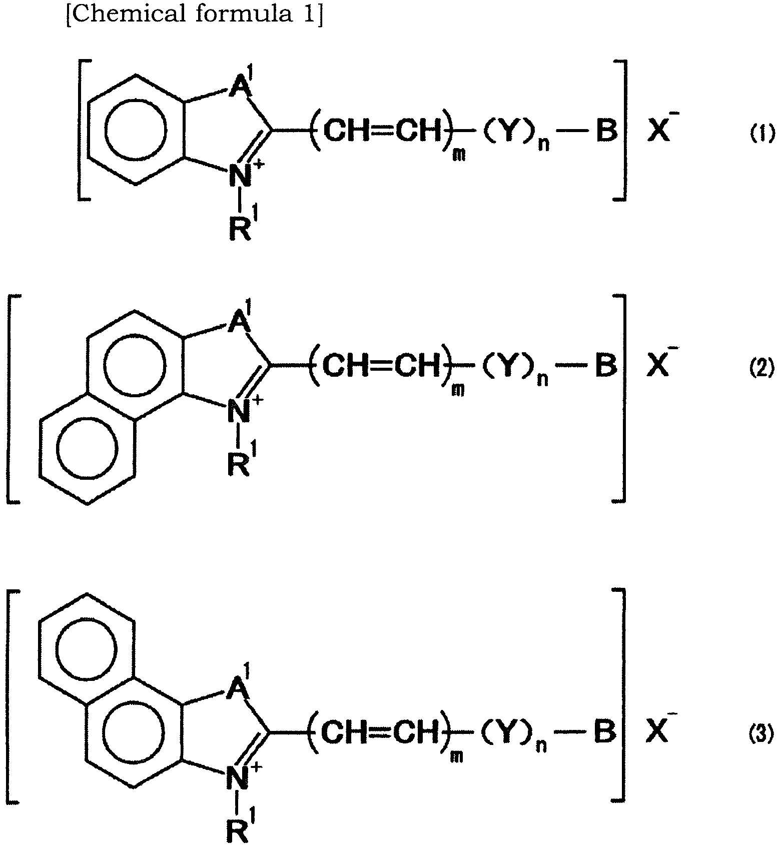 (wherein, A¹ is -O-, -S-, -Se- or -C(CH₃)₂-, R¹ is a lower alkyl group, X is a halogen or perchloric acid, Y is -CH= or -NH-, m is 1 or 2, n is 0 or 1, and B is represented by the following formula: 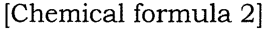 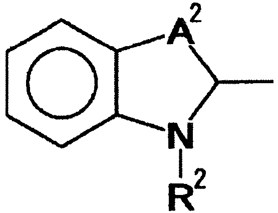 (wherein, A² is -O-, -S- or -C(CH₃)₂-, R² is a lower alkyl group or a phenyl group substituted with two lower alkoxy groups or by one di-lower alkylamino group (this lower alkyl may be substituted by a cyano group))

In the above formulas (1) to (3), A¹ and A² may be the same or different from each other. Also, R¹ and R² may be the same or different from each other.

The lower alkyl group means an alkyl group having 1 to 6 carbons, and examples of the same include methyl, ethyl, propyl, butyl, isobutyl, pentyl, hexyl and the like. Examples of the halogen atom of X include fluorine, chlorine, bromine and iodine. The phenyl group substituted with two lower alkoxy groups in B means a phenyl group substituted with two alkoxy groups having 1 to 3 carbons, preferably alkoxy groups having 1 or 2 carbons, e.g., a methoxy group and ethoxy groups. Specific examples include a 2,6-dimethoxyphenyl group, and a 2,6-diethoxyphenyl group. The phenyl group substituted with a di-lower alkylamino group in B (the lower alkyl group may be substituted with a cyano group) means a phenyl group substituted with an alkylamino group having 1 to 3 carbons, preferably an alkylamino group having 1 or 2 carbons. The alkyl group used herein may be substituted by a cyano group, and examples of the same include methyl, ethyl, cyanomethyl, cyanoethyl and the like. Preferable examples of the phenyl group substituted with a di-lower alkylamino group (the lower alkyl may be substituted with a cyano group) include a 4-dimethylaminophenyl group, a 4-diethylaminopheyl group, and a 4-(cyanoethylmethylamino)phenyl group.

Examples of the dye that binds to minor grooves of DNA include Hoechst 33342, Hoechst 33258 and 4',6-diamidino-2-phenylindole dihydrochloride (DAPI).

The first reagent may contain one fluorescent dye or two or more fluorescent dyes. The concentration of the fluorescent dye in the first reagent is desirably set so that the fluorescent dye is contained in such a final concentration capable of appropriately staining at least sperms and yeast-like fungi in the measurement sample prepared in the manner as described above. The final concentration in the measurement sample may be appropriately set depending on the kind of the fluorescent dye. For example, when thiazole orange is used as the fluorescent dye, the final concentration in the measurement sample is more than or equal to 0.1 µg/mL and less than or equal to 200 µg/mL, preferably more than or equal to 0.5 µg/mL and less than or equal to 50 µg/mL.

The first reagent can be obtained by dissolving the fluorescent dye in an appropriate solvent. The solvent is not particularly limited as far as it is an aqueous solvent capable of dissolving the fluorescent dye and examples thereof include water, a water-soluble organic solvent and a mixture thereof. Among these, a water-soluble organic solvent is particularly preferable. Examples of the water-soluble organic solvent include lower alcohols having 1 to 3 carbon atoms, ethylene glycol, dimethylsulfoxide (DMSO) and the like.

The second reagent can be obtained by dissolving acetic acid and/or a salt thereof as an antiseptic agent in an appropriate solvent. In general, an antiseptic agent is added to the reagent for analyzing urine sample for the purpose of storing the reagent stably. The present inventor has found that by using acetic acid and/or a salt thereof as an antiseptic agent to be added to the reagent for analysis, it is possible to inhibit a change in the form of yeast-like fungi and to vary a stainability of the yeast-like fungi, thereby detecting sperms and yeast-like fungi more accurately. Acetic acid for use in preparation of the second reagent may be a solution or solid (glacial acetic acid). When the solvent is water or contains water, the second reagent may be prepared by using acetic anhydride in place of acetic acid.

In the present embodiment, the kind of acetate is not particularly limited as far as it has a bacteriostatic action on bacteria or fungi such as mold, however, salts of alkali metal or alkali earth metal are preferable For example, sodium acetate, potassium acetate, calcium acetate, magnesium acetate and so on are recited, and among these, sodium acetate is particularly preferable. Also, sodium diacetate (CAS No. 126-96-5) obtainable by mixing acetic acid and sodium acetate in 1 : 1 may be used as the antiseptic agent. The second reagent may contain one acetate or two or more acetates.

The concentration of acetic acid or acetate in the second reagent is not particularly limited as far as the effect as the antiseptic agent is obtained. For example, when acetic acid is used, the concentration in the reagent is typically more than or equal to 100 ppm and less than or equal to 3000 ppm, preferably more than or equal to 200 ppm and less than or equal to 1000 ppm. The concentration of acetic acid in the reagent can be calculated on the basis of the purity of acetic acid used in preparation.

The solvent used in the second reagent is not particularly limited as far as it can dissolve the acetic acid and/or a salt thereof and may include, for example, water, a water-soluble organic solvent and a mixture thereof. Examples of the water-soluble organic solvent include lower alcohols having 1 to 3 carbons, ethylene glycol and DMSO. In the present embodiment, water is particularly preferred.

In the present embodiment, preferably, the second reagent further contains a surfactant for lysing erythrocytes and damaging urinary particles having nucleic acid so as to be permeable to the fluorescent dye. Such a surfactant can be selected from cationic surfactants and nonionic surfactants that are known in the art. The second reagent may contain one surfactant or two or more surfactants. When two or more surfactants are contained, the combination thereof may be optionally selected from the cationic surfactants and/or the nonionic surfactants.

In the present embodiment, as the cationic surfactant, at least one selected from quaternary ammonium salt type surfactants and pyridinium salt type surfactants may be used. The quaternary ammonium salt type surfactant may include, for example, the surfactants having 9 to 30 carbon atoms in total represented by the following formula (I).

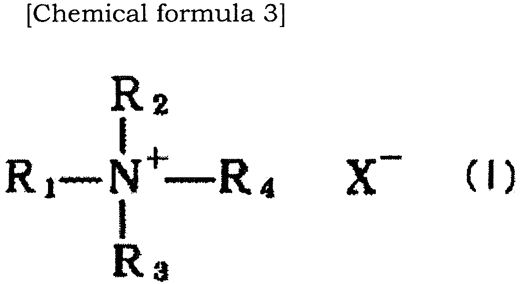

In the above formula (I), R₁ is an alkyl or alkenyl group having 6 to 18 carbon atoms; R₂ and R₃ are the same or different from each other and are respectively an alkyl or alkenyl group having 1 to 4 carbon atoms; R₄ is an alkyl or alkenyl group having 1 to 4 carbon atoms, or a benzyl group; and X- is a halogen ion.

In the above formula (I), R₁ is preferably an alkyl group or an alkenyl group having 6, 8, 10, 12 or 14 carbon atoms and is particularly preferably a linear alkyl group. More specifically, R₁ may include an octyl group, a decyl group and a dodecyl group. R₂ and R₃ are preferably a methyl group, an ethyl group and a propyl group. R₄ is preferably a methyl group, an ethyl group and a propyl group.

The pyridinium salt type surfactant may include, for example, the surfactants represented by the following formula (II).

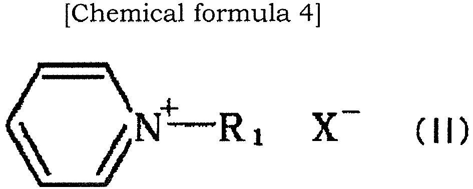

In the above formula (II), R₁ is an alkyl or alkenyl group having 6 to 18 carbon atoms; and X- is a halogen ion.

In the above formula (II), R₁ is preferably an alkyl or alkenyl group having 6, 8, 10, 12 or 14 carbon atom and is particularly preferably a linear alkyl group. More specifically, R₁ include an octyl group, a decyl group and a dodecyl group.

Specific examples of the cationic surfactant include dodecyltrimethylammonium bromide, decyltrimethylammonium bromide, dodecyltrimethylammonium chloride, octyltrimethylammonium bromide, octyltrimethylammonium chloride, myristyltrimethylammonium bromide, myristyltrimethylammonium chloride and dodecylpyridinium chloride. Among these, dodecyltrimethylammonium bromide (DTAB) is particularly preferable.

In the present embodiment, as the nonionic surfactant, the polyoxyethylene-based nonionic surfactants represented by the following formula (III) are preferably used.
[Chemical formula 5]

R₁-R₂-(CH₂CH₂O)ₙ-H (III)

In the above formula (III), R₁ is an alkyl, alkenyl or alkynyl group having 8 to 25 carbon atoms; R₂ is -O-, -COO- or 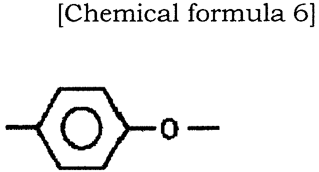 ; and n is an integer of 10 to 50.

Specific examples of the nonionic surfactant include polyoxyethylene alkyl ether, polyoxyethylene sterol, polyoxyethylene castor oil, polyoxyethylene sorbit fatty acid ester, polyoxyethylene alkyl amine and polyoxyethylene polyoxypropylene alkyl ether.

The concentration of the surfactant in the second reagent is desirably set so that the surfactant is contained in a final concentration that lyses erythrocytes and can damage urinary particles (at least sperms and yeast-like fungi) having nucleic acid to such a degree that the urinary particles become permeable to the fluorescent dye, in the measurement sample prepared in the manner as described above. The final concentration of the surfactant in the measurement sample may be appropriately set depending on the kind of the surfactant. For example, when DTAB is used as the surfactant, the final concentration in the measurement sample is more than or equal to 100 ppm and less than or equal to 2500 ppm, preferably more than or equal to 500 ppm and less than or equal to 2000 ppm.

In the present embodiment, in order to facilitate hemolysis of erythrocytes in the urine sample, pH of the second reagent can be adjusted in the range of higher than or equal to 3 and lower than or equal to 6, preferably in a range of higher than or equal to 5 and lower than or equal to 6. Therefore, in order to keep pH constant, the second reagent may contain a buffer. The buffer is not particularly limited as far as it has the buffering action in the aforementioned pH ranges and examples thereof include a combination of citric acid and a salt thereof, a combination of phosphoric acid and a salt thereof and the like. When a combination of acetic acid and sodium acetate or potassium acetate is contained as the antiseptic agent, the combination acts as an acetate buffer, and thus it is not necessary to further add a buffer to the second reagent.

Some urine samples contain amorphous salts such as ammonium phosphate, magnesium phosphate and calcium carbonate. In the present embodiment, the second reagent may contain a chelating agent for reducing the influence of these amorphous salts. The chelating agent is not particularly limited as far as it is a chelating agent capable of removing amorphous salts, and can be appropriately selected from calcium removing agents and magnesium removing agents that are known in the art. Specific examples include ethylenediaminetetraacetate (EDTA salt), CyDTA, DHEG, DPTA-OH, EDDA, EDDP, GEDTA, HDTA, HIDA, Methyl-EDTA, NTA, NTP, NTPO, EDDPO and the like, and among these, EDTA salt is particularly preferable.

The concentration of the chelating agent in the second reagent is desirably set so that the chelating agent is contained in such a final concentration that can reduce the influence of the amorphous salts in the measurement sample prepared in the manner as described above. The final concentration in the measurement sample may be appropriately set depending on the kind of the chelating agent. For example, when EDTA 2 potassium (EDTA-2K) is used as the chelating agent, the final concentration in the measurement sample is more than or equal to 100 mM and less than or equal to 300 mM, preferably more than or equal to 150 mM and less than or equal to 250 mM.

The osmotic pressure of urine is known to distribute in a wide range of 50 to 1300 mOsm/kg. When the osmotic pressure is too low or too high in the measurement sample, there is a fear that cells such as leukocytes are damaged. An appropriate osmotic pressure in the measurement sample is more than or equal to 100 mOsm/kg and less than or equal to 600 mOsm/kg, preferably more than or equal to 150 mOsm/kg and less than or equal to 500 mOsm/kg. When the osmotic pressure of the urine is too high, the osmotic pressure can be appropriately adjusted by diluting the urine with water or the second reagent. On the other hand, when the osmotic pressure of the urine is too low, the second reagent may contain an osmotic pressure compensating agent. The osmotic pressure compensating agent may include inorganic salts, organic salts, saccharides and the like. Examples of the inorganic salts include sodium chloride, sodium bromide and the like. Examples of the organic salts include sodium propionate, potassium propionate, ammonium propionate oxalate and the like. Examples of the saccharides include sorbitol, glucose, mannitol and the like.

In the method of the present embodiment, a step of obtaining optical information by irradiating urinary particles contained in the measurement sample obtained in the above preparation step with light is conducted.

The obtention step is desirably conducted by a flow cytometer. In the measurement by a flow cytometer, by irradiating stained urinary particles with light when the particles pass through a flow cell, the optical information can be obtained as signals emitted from the particles. As such optical information, scattered light information and fluorescence information are preferable.

The scattered light information is not particularly limited as far as it is the information of scattered light that can be measured on a conventional commercially available flow cytometer. The scattered light information may include, for example, intensity and waveform information of scattered light such as forward scattered light (e.g. light receiving angle of about 0 to 20 degrees) and side scattered light (light receiving angle of about 90 degrees). More specifically, the scattered light information includes an intensity of scattered light, a pulse width of scattered light, an integral value of scattered light and the like. It is known in the art that side scattered light reflects the internal information of cells such as nuclei and granules, and forward scattered light reflects the information of the size of cells. In the embodiment, it is preferred to use the information of forward scattered light.

The fluorescence information is not particularly limited as far as it is the information obtained by irradiating the stained urinary particles with excitation light having an appropriate wavelength and measuring the excited fluorescence. The fluorescence information may include, for example, an intensity and a waveform of fluorescence. More specifically, the fluorescence information includes an intensity of fluorescence, a pulse width of fluorescence, an integral value of fluorescence and the like. The fluorescence is emitted from nucleic acid or the like in the particles stained by the fluorescent dye contained in the first reagent. The receiving wavelength can be appropriately selected depending on the fluorescent dye contained in the first reagent.

In the present embodiment, the light source of the flow cytometer is not particularly limited, and a light source having a suitable wavelength for excitation of the fluorescent dye can be appropriately selected. For example, a red semiconductor laser, a blue semiconductor laser, an argon laser, a He-Ne laser, a mercury arc lamp or the like may be used. In particular, a semiconductor laser is suitable because it is very low in price compared with a gas laser.

In the method of the present embodiment, a step of detecting at least sperms and yeast-like fungi as urinary particles on the basis of the optical information obtained in the obtention step is conducted. The term "detection" embraces not only finding the existence of the urinary particles in the measurement sample but also classifying and counting the urinary particles.

In the present embodiment, detection of the urinary particles is preferably conducted by preparing a scattergram having two axes of scattered light information and fluorescence information, and analyzing the obtained scattergram by using appropriate analysis software. For example, when a scattergram is prepared having a fluorescent intensity on the X-axis and a forward scattered light intensity on the Y-axis, respective groups (clusters) appear on the scattergram in accordance with the particle size and the stainability (nucleic acid content) of the respective urinary particles. In the method of the present embodiment, at least sperms and yeast-like fungi can be detected as two groups appearing in different areas.

In the present embodiment, besides sperms and yeast-like fungi, Trichomonas, leukocytes, epithelial cells, atypical cells and bacteria can be also detected as groups that appear in different areas from each other.

Since sperms, Trichomonas and yeast-like fungi have smaller amounts of nucleic acid compared with leukocytes, epithelial cells and atypical cells, they have lower stainability to a staining dye compared with leukocytes, epithelial cells and atypical cells. Therefore, it is possible to classify sperms, Trichomonas and yeast-like fungi, differently from leukocytes, epithelial cells and atypical cells on the basis of the fluorescence intensity. Since bacteria have very small sizes and contain small amounts of nucleic acid compared with sperms, Trichomonas, yeast-like fungi, leukocytes, epithelial cells and atypical cell, they have lower stainability to a staining dye compared with sperms, Trichomonas, yeast-like fungi, leukocytes, epithelial cells and atypical cells. Therefore, it is possible to classify bacteria differently from sperms, Trichomonas, yeast-like fungi, leukocytes, epithelial cells and atypical cells on the basis of the fluorescence intensity.

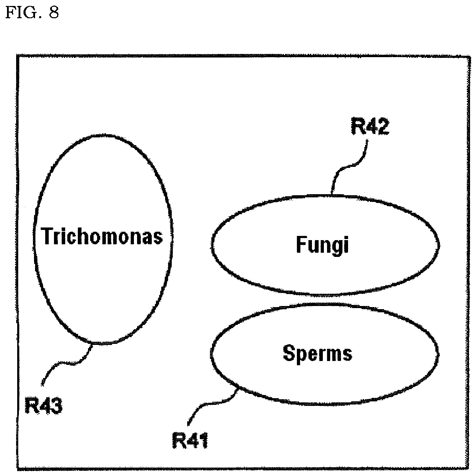

Fig. 8 is a schematic diagram of scattergram showing distributions of sperms, Trichomonas and yeast-like fungi. Particles contained in the area R41 shown in Fig. 8 are detected as sperms, and the number thereof is counted. Also, particles contained in the area R42 shown in Fig. 8 are detected as yeast-like fungi, and the number thereof is counted. Further, particles contained in the area R43 shown in Fig. 8 are detected as Trichomonas, and the number thereof is counted.

The area where the fluorescent intensity distributes differs between sperms and yeast-like fungi. This is because the amount of nucleic acid in sperms is larger than the amount of nucleic acid in yeast-like fungi. Also, the area where the forward scattered light intensity distributes differs between sperms and fungi, and Trichomonas. This is because the size of Trichomonas is larger than the sizes of sperms and fungi. Therefore, it is possible to classify sperms, Trichomonas and yeast-like fungi on the basis of the fluorescence intensity and the forward scattered light intensity.

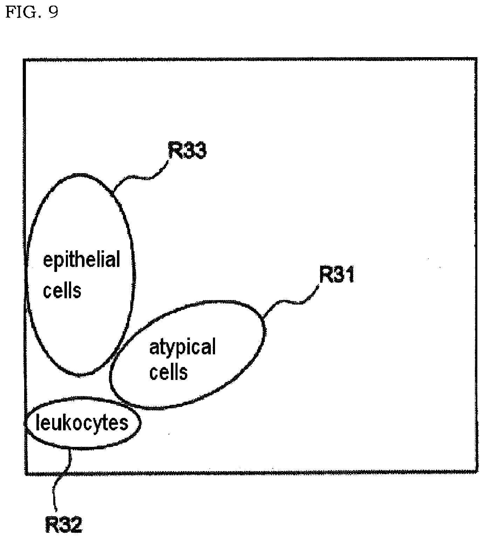

Fig. 9 is a schematic diagram of scattergram showing distributions of leukocytes, atypical cells and epithelial cells. Particles contained in the area R31 shown in Fig. 9 are detected as atypical cells, and the number thereof is counted. Also, particles contained in the area R32 shown in Fig. 9 are detected as leukocytes, and the number thereof is counted. Further, particles contained in the area R33 shown in Fig. 9 are detected as epithelial cells, and the number thereof is counted.

The area where the fluorescent intensity distributes differs between leukocytes and epithelial cells, and atypical cells. This is because there is almost no difference in the amount of nucleic acid between leukocytes and epithelial cells, and the amount of nucleic acid is larger in atypical cells than in leukocytes and epithelial cells, and the fluorescence intensity reflects the amount of nucleic acid. Also, the area where the forward scattered light intensity distributes differs between leukocytes and epithelial cells. This is because epithelial cells have a larger size than leukocytes, and the forward scattered light intensity reflects the size of particles. Therefore, it is possible to classify leukocytes, epithelial cells and atypical cells on the basis of the fluorescence intensity and the forward scattered light intensity.

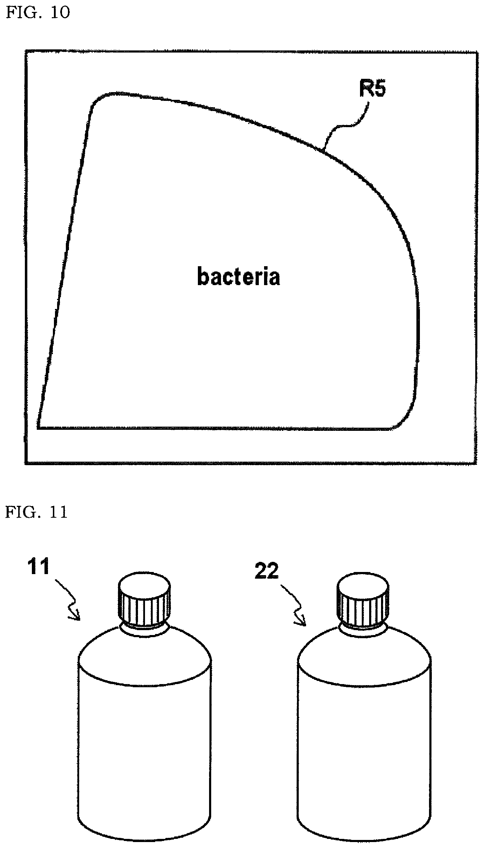

Fig. 10 is a schematic diagram of scattergram showing distribution of bacteria. Particles contained in the area R5 shown in Fig. 10 are detected as bacteria, and the number thereof is counted.

An analysis software provides windows which enclose the respective groups on the scattergram and allows count the number of particles in each window.

### [Use of a reagent kit for analysis of urine sample]

In the use of a reagent kit for analysis of urine sample of the present embodiment (hereinafter, simply referred to as "reagent kit") the reagent kit is a reagent kit for detecting sperms and yeast-like fungi in a urine sample by differentiating therebetween. The reagent kit includes a first reagent containing a fluorescent dye capable of staining nucleic acid, and a second reagent containing acetic acid and/or a salt thereof as an antiseptic agent and a surfactant for lysing erythrocytes and damaging urinary particles having nucleic acid so as to be permeable to the fluorescent dye.

The details of the first reagent included in the reagent kit are the same as those described for the first reagent used in the method for analyzing urine sample of the present embodiment.

The details of the second reagent included in the reagent kit are the same as those described for the second reagent used in the method for analyzing urine sample of the present embodiment. except that acetic acid and/or a salt thereof as an antiseptic agent and a surfactant are contained. The details of acetic acid and/or a salt thereof as the antiseptic agent and the surfactant for use in the reagent kit are the same as those described in the explanation of the method for analyzing urine sample of the present embodiment.

In the present embodiment, it is preferable to store the first reagent and the second reagent in separate containers so as to provide a two-reagent type reagent kit comprising these reagents. Fig. 11 shows one example of a reagent kit of the present embodiment including a first reagent 11 stored in a container, and a second reagent 22 stored in a container.

The scope of the present invention also encompasses use of a reagent kit comprising a first reagent containing a fluorescent dye capable of staining nucleic acid, and a second reagent containing acetic acid and/or a salt thereof as an antiseptic agent and a surfactant for lysing erythrocytes and damaging urinary particles having nucleic acid so as to be permeable to the fluorescent dye, for detecting sperms and yeast-like fungi in a urine sample by differentiating therebetween.

The present invention is further described in detail hereinafter by way of examples which do not limit the present invention.

### EXAMPLES

### Example 1

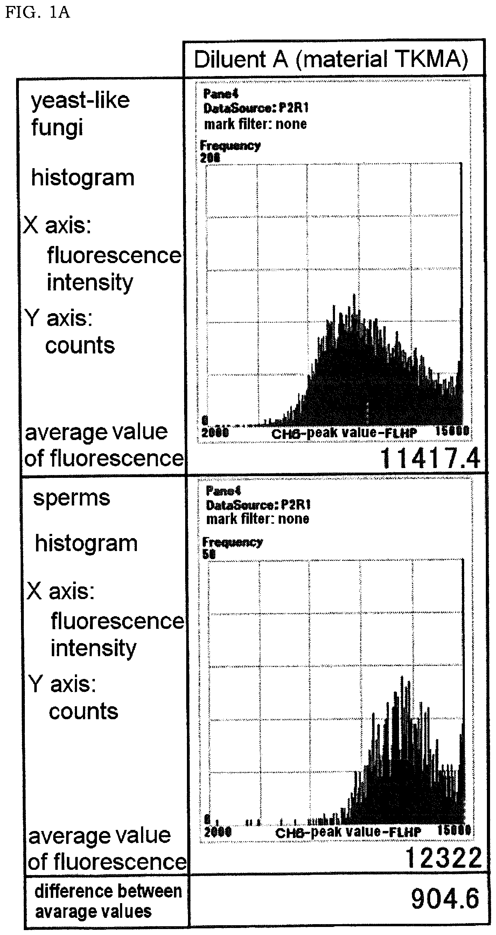

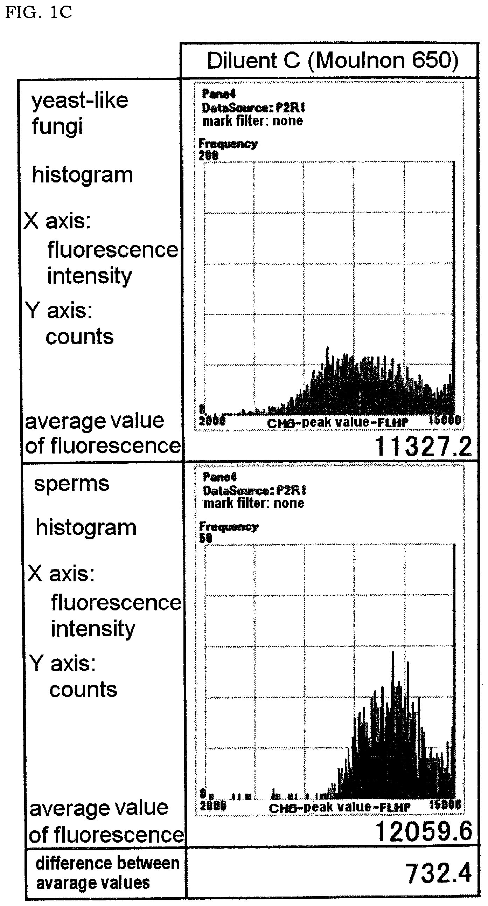

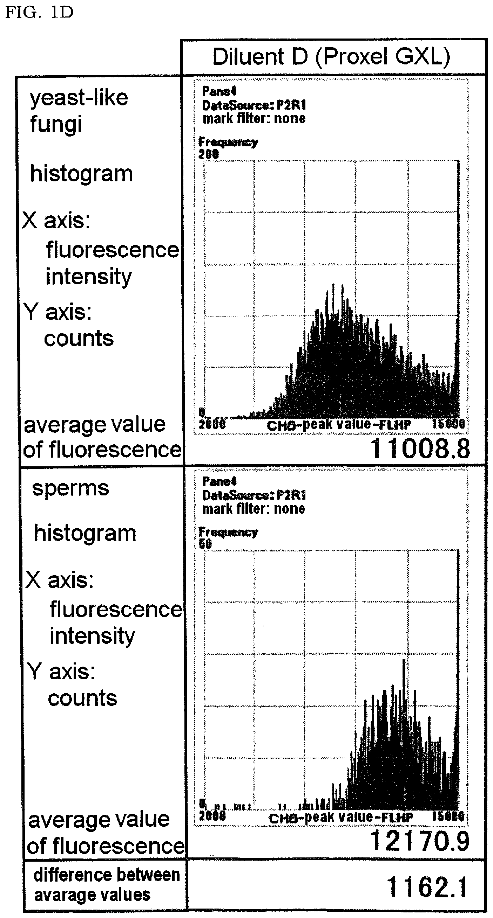

In Example 1, for examining an antiseptic agent that is suited for discrimination between sperms and yeast-like fungi by a flow cytometer, the discriminability was compared by using reagents for analysis containing various antiseptic agents. The discriminability was evaluated on the basis of the difference between the average measurement for the sample containing sperms and the average measurement for the sample containing yeast-like fungi.
(1) Samples
   - Sample containing sperms
      Sperms (200 µL) collected from a healthy volunteer were suspended in a saline (50 mL) to prepare a sample containing sperms.
   - Sample containing yeast-like fungi
      Cultured *Candida albicans (C. albicans*: obtained from ATCC) was suspended in a saline to a concentration of 1.0 × 10⁶ cells/mL to prepare a sample containing yeast-like fungi.
(2) Reagent
   - Staining reagent
      As a fluorescent dye capable of staining nucleic acid, NK-9536 (thiazole orange: HAYASHIBARA CO., LTD.) was dissolved in ethylene glycol (NACALAI TESQUE, INC.) to a concentration of 0.20 mg/mL to prepare a staining reagent.
   - Diluting reagent
      As a diluting reagent, diluents A to D having the following compositions were prepared. The diluents A to D respectively contain material TKMA which is pyridine-based, acetic acid, Moulnon 650 which is triazine-based, and Proxel GXL which is isothiazoline-based as an antiseptic agent. For the diluents A to D, water filtrated through a reverse osmosis membrane was used as a solvent. The purity of acetic acid used in preparing the diluent B is 99%.
      Diluent A (pH 5.6): DTAB (1250 ppm)(Tokyo Chemical Industry Co., Ltd.), EDTA-2K (25 mM)(Chubu Chelest Co., Ltd.) and material TKMA (1000 ppm: API CORPORATION)
      Diluent B (pH 5.6): DTAB (1250 ppm), EDTA-2K (25 mM) and acetic acid (1000 ppm)(Wako Pure Chemical Industries, Ltd.)
      Diluent C (pH 5.6): DTAB (1250 ppm), EDTA-2K (25 mM) and Moulnon 650 (1000 ppm)(KATAYAMA CHEMICAL INDUSTRIES CO., LTD.)
      Diluent D (pH 5.6): DTAB (1250 ppm), EDTA-2K (25 mM) and Proxel GXL (1000 ppm)(Arch Chemicals, Inc.)
   - Sheath solution
      As a sheath solution, UF-F sheath (Sysmex Corporation) was used.
(3) Measurement and result
   The samples were measured by using a flow cytometer UF-1000i (Sysmex Corporation). Specific steps of measurement by this flow cytometer are as follows. First, a sample (200 µL) and a diluent (580 µL) that was preliminarily warmed to 42°C were mixed and allowed to react at 42°C for 7 seconds. Then the obtained mixture and a staining reagent (20 µL) were mixed and allowed to react at 42°C for 3 seconds to prepare a measurement sample. Then the obtained measurement sample was irradiated with light to obtain the fluorescence intensity. The flow cytometer had a light source of a semiconductor laser having an excitation wavelength of 488 nm. The obtained results are shown in Figs. 1A to 1D.

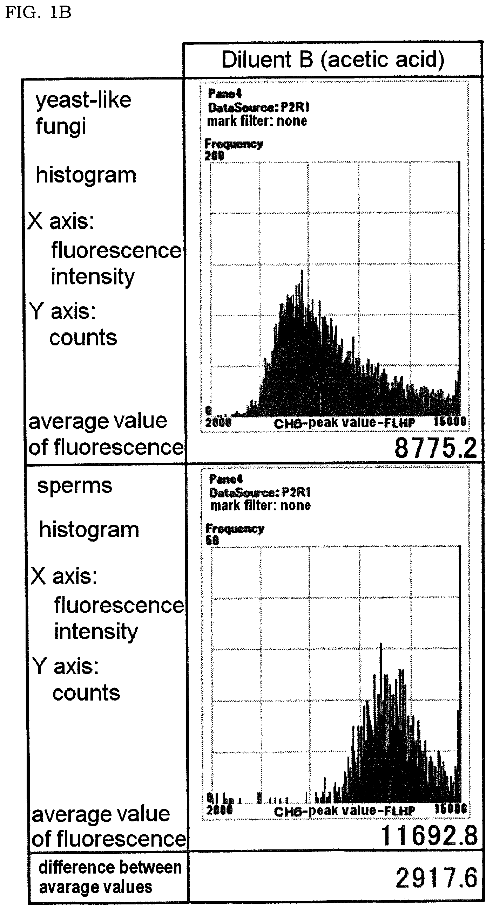

As shown in Figs. 1A to 1D, for the sample containing sperms, no significant difference was observed in the average value of the fluorescence intensity and the histogram in any case where any of the antiseptic agents was used. For the sample containing yeast-like fungi, the average value of the fluorescence intensity in the case of using an antiseptic agent other than acetic acid was close to the average value for the sample containing sperms (see Figs. A, C and D). In contrast, when acetic acid was used as an antiseptic agent, the average value of the fluorescence intensity for the sample containing yeast-like fungi was lower than that when other antiseptic agent was used (see Fig. 1B). Therefore, it was found that the difference between the average measurement for the sample containing sperms and the average measurement for the sample containing yeast-like fungi is significantly larger when acetic acid was used than when an antiseptic agent other than acetic acid was used. These results reveal that when a diluent containing acetic acid as an antiseptic agent is used, the discriminability between sperms and yeast-like fungi is higher in comparison with the case where a diluent containing a conventionally used and commercially available antiseptic agent is used.

### Example 2

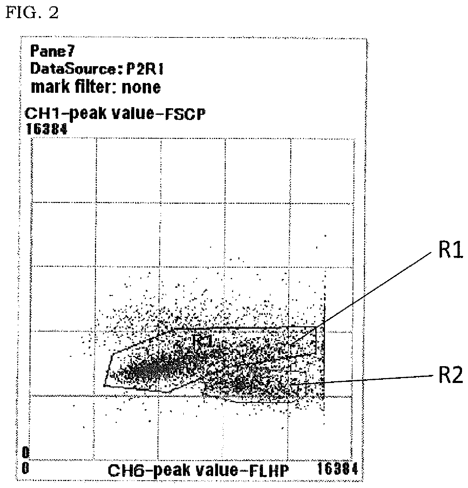

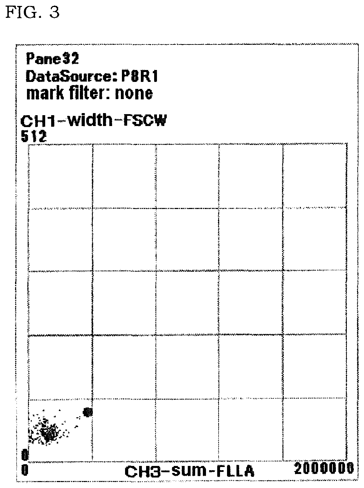

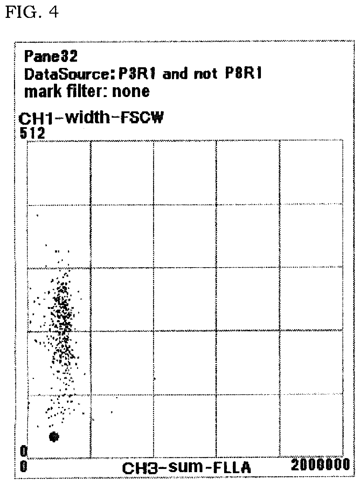

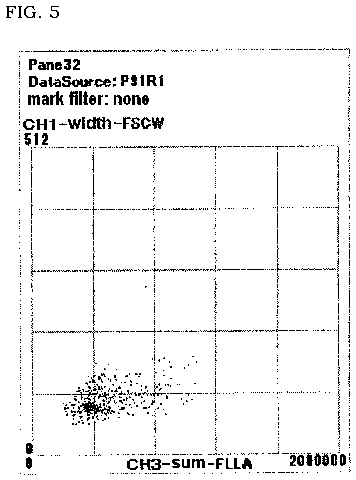

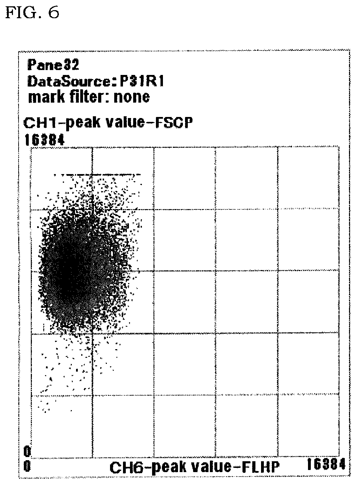

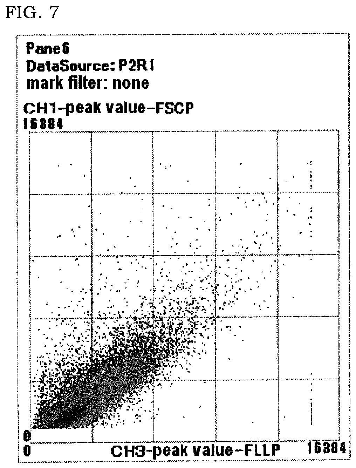

In Example 2, using a staining reagent and a diluting reagent containing acetic acid as an antiseptic agent, a sample containing sperms and yeast-like fungi was measured with a flow cytometer to evaluate the discriminability. The discriminability was evaluated on the basis of a scattergram prepared from the obtained fluorescence intensity and scattered light intensity.
(1) Samples
   - Sample containing sperms and yeast-like fungi
      Sperms (200 µL) collected from a healthy volunteer was suspended in a saline (50 mL) to prepare a suspension containing sperms. In this suspension, cultured *Candida albicans* was suspended to a concentration of 5.0 × 10⁵ cells/mL to prepare a sample containing sperms and yeast-like fungi.
   - Sample containing leukocytes
      Leukocytes were suspended in a saline to prepare a sample containing leukocytes.
   - Sample containing epithelial cells
      Epithelial cells were suspended in a saline to prepare a sample containing epithelial cells.
   - Sample containing atypical cells
      Atypical cells were suspended in a saline to prepare a sample containing atypical cells.
   - Sample containing Trichomonas
      Trichomonas was suspended in a saline to prepare a sample containing Trichomonas.
   - Sample containing bacterium
      A bacterium was suspended in a saline to prepare a sample containing a bacterium.
(2) Reagent
   As a staining reagent, the same staining reagent as in Example 1 was used. As a diluting reagent, the same diluent B as in Example 1 was used. As a sheath solution, the same UF-F sheath (Sysmex Corporation) as in Example 1 was used.
(3) Measurement and result
   The samples were measured by using a flow cytometer UF-1000i (Sysmex Corporation). The measurement sample was prepared in the same manner as in Example 1. Then the obtained measurement sample was irradiated with light to obtain the fluorescence intensity and the forward scattered light intensity. The flow cytometer had a light source of a semiconductor laser having an excitation wavelength of 488 nm. The scattergrams were prepared based on these measurements. The results are shown in Figs. 2 to 7. In Figs. 2 to 7, X axis (horizontal axis) indicates the fluorescence intensity and Y axis (vertical axis) indicates the forward scattered light intensity. Fig. 2 reveals that by using a diluting reagent containing acetic acid as an antiseptic agent, it is possible to fractionate the scattergram into an area of yeast-like fungi (R1) and an area of sperms (R2) on the basis of the fluorescence intensity and the forward scattered light intensity. Similarly, Figs. 3 to 7 reveal that by using a diluting reagent containing acetic acid as an antiseptic agent, it is possible to detect leukocytes, epithelial cells, atypical cells, Trichomonas and bacteria in the scattergram on the basis of the fluorescence intensity and the forward scattered light intensity.

### Example 3

In this example, the antimicrobial activity of acetic acid against various bacteria was evaluated. As a control, a saline was used. The antimicrobial activity was evaluated on the basis of the number of colonies of the bacterium on the solid culture medium.
(1) Bacteria used
   - *Escherichia coli (E. coli:* obtained from ATCC)
   - *Staphylococcus aureus (S. aureus:* obtained from ATCC)
   - *Pseudomonas aeruginosa (P. aeruginosa:* obtained from ATCC)
(2) Reagent
   - Acetic acid aqueous solution (1000 ppm)
   - Saline
(3) Evaluation of antimicrobial activity

To each of an acetic acid aqueous solution and a saline, various species of bacteria were added to a concentration of 1 × 10⁵ cells/mL to obtain bacterial suspensions. Each 100 µL was taken from the obtained bacterial suspensions, and these were taken as samples at day 0 of culture. The remaining bacterial suspensions were cultured for 7 days at 37°C for E. *coli* and *S*. *aureus* and at 25°C for *P. aeruginosa,* and each 100 µL was taken from the obtained bacterial suspensions as samples at day 7 of culture. Each 100 µL of the sample was coated on a heart infusion agar culture medium, and cultured at the respective culture temperatures. The culture time was 20 hours for *E. coli,* 24 hours for *S*. *aureus* and 48 hours for P. *aeruginosa.* After culturing, the number of colonies on the agar culture medium was counted, and the bacterial number (CFU/mL) was calculated. The result is shown in Table 1.

**[Table 1]**

| Bacterial species | Reagent | Bacterial number (CFU/mL) | |
|---|---|---|---|
| | | At day 0 | At day 7 |
| *E. coli* | 1000 ppm acetic acid | 1000 | 0 |
| | Saline | 1000 | 66080 |
| *S*. *aureus* | 1000 ppm acetic acid | 1000 | 0 |
| | Saline | 1000 | 1630 |
| *P. aeruginosa* | 1000 ppm acetic acid | 1000 | 0 |
| | Saline | 1000 | 3740 |

Table 1 reveals that any of E. coli, S. aureus and P. aeruginosa cannot be proliferated in the 1000 ppm acetic acid aqueous solution even after culturing for 7 days. Therefore, it was demonstrated that by using acetic acid as an antiseptic agent for a diluting reagent, a sufficient antiseptic effect on these bacteria is obtained.

The present application relates to Japanese patent application No. 2014-39283 filed on February 28, 2014, and the entirety of these claims, description, figures and abstract are incorporated into the present specification by reference.

### REFERENCE SIGNS LIST

11: First reagent
22: Second reagent

## Claims

1. A method for analyzing a urine sample comprising the steps of:
preparing a measurement sample by mixing the urine sample, a first reagent containing a fluorescent dye capable of staining nucleic acid and, a second reagent containing acetic acid and/or a salt thereof as an antiseptic agent,
obtaining optical information by irradiating urinary particles contained in the measurement sample obtained in the preparation step with light, and
detecting at least sperms and yeast-like fungi as urinary particles on the basis of the optical information obtained in the obtention step.

2. The method for analyzing the urine sample according to claim 1, wherein the optical information is scattered light information and fluorescence information.

3. The method for analyzing the urine sample according to claim 1, wherein the second reagent further contains a surfactant for lysing erythrocytes and damaging urinary particles having nucleic acid so as to be permeable to the fluorescent dye.

4. The method for analyzing the urine sample according to claim 3, wherein the surfactant is at least one selected from a cationic surfactant and a nonionic surfactant.

5. The method for analyzing the urine sample according to claim 4, wherein the cationic surfactant is selected from a quaternary ammonium salt type surfactant and a pyridinium salt type surfactant; and
wherein the nonionic surfactant is selected from a polyoxyethylene-based nonionic surfactant.

6. The method for analyzing the urine sample according to claim 4, wherein the cationic surfactant is dodecyltrimethylammonium bromide.

7. The method for analyzing the urine sample according to claim 1, wherein pH of the second reagent is higher than or equal to 3 and lower than or equal to 6.

8. The method for analyzing the urine sample according to claim 1, wherein the second reagent further contains a chelating agent.

9. The method for analyzing the urine sample according to claim 8, wherein the chelating agent is ethylenediaminetetraacetate (EDTA salt).

10. Use of a reagent kit for analysis of urine sample for detecting sperms and yeast-like fungi in the urine sample by differentiating therebetween, wherein the reagent kit comprises
a first reagent containing a fluorescent dye capable of staining nucleic acid, and
a second reagent containing acetic acid and/or a salt thereof as an antiseptic agent and a surfactant for lysing erythrocytes and damaging urinary particles having nucleic acid so as to be permeable to the fluorescent dye.

11. The use of the reagent kit for analysis of urine sample according to claim 10, wherein the surfactant is at least one selected from a cationic surfactant and a nonionic surfactant.

12. The use of the reagent kit for analysis of urine sample according to claim 11, wherein the cationic surfactant is a quaternary ammonium salt type surfactant and a pyridinium salt type surfactant; and
wherein the nonionic surfactant is a polyoxyethylene-based nonionic surfactant.

13. The use of the reagent kit for analysis of urine sample according to claim 11, wherein the cationic surfactant is dodecyltrimethylammonium bromide.

14. The use of the reagent kit for analysis of urine sample according to claim 10, wherein pH of the second reagent is higher than or equal to 3 and lower than or equal to 6.

15. The use of the reagent kit for analysis of urine sample according to claim 10, wherein the second reagent further contains a chelating agent.

16. The use of the reagent kit for analysis of urine sample according to claim 15, wherein the chelating agent is ethylenediaminetetraacetate (EDTA salt).

## Patentansprüche

1. Verfahren zum Analysieren einer Urinprobe, umfassend die Schritte:
Herstellung einer Messprobe durch Mischen der Urinprobe, einem ersten Reagens, das einen Fluoreszenzfarbstoff enthält, der in der Lage ist, Nukleinsäure zu färben, und einem zweiten Reagens, das Essigsäure und/oder ein Salz davon als ein antiseptisches Mittel enthält,
Erhalten von optischer Information durch Bestrahlung von Urinpartikeln, die in der im Herstellungsschritt erhaltenen Messprobe enthalten sind, mit Licht, und
Nachweis von zumindest Spermien und hefeartigen Pilzen als Urinpartikel basierend auf der optischen Information, die im Schritt des Erhaltens erhalten wurde.

2. Verfahren zum Analysieren der Urinprobe gemäß Anspruch 1, wobei die optische Information Streulichtinformation und Fluoreszenzinformation ist.

3. Verfahren zum Analysieren der Urinprobe gemäß Anspruch 1, wobei das zweite Reagens weiter ein Tensid zum Lysieren von Erythrozyten und Beschädigen von Urinpartikeln mit Nukleinsäure, so dass sie für den Fluoreszenzfarbstoff durchlässig sind, enthält.

4. Verfahren zum Analysieren der Urinprobe gemäß Anspruch 3, wobei das Tensid zumindest eines, ausgewählt aus einem kationischen Tensid und einem nicht-ionischen Tensid, ist.

5. Verfahren zum Analysieren der Urinprobe gemäß Anspruch 4, wobei das kationische Tensid ausgewählt ist aus einem Tensid vom Typ eines quaternären Ammoniumsalzes und einem Tensid vom Typ eines Pyridiniumsalzes; und
wobei das nicht-ionische Tensid aus einem Polyoxyethylen-basierten nicht-ionischen Tensid ausgewählt ist.

6. Verfahren zum Analysieren der Urinprobe gemäß Anspruch 4, wobei das kationische Tensid Dodecyltrimethylammoniumbromid ist.

7. Verfahren zum Analysieren der Urinprobe gemäß Anspruch 1, wobei der pH-Wert des zweiten Reagens höher als oder gleich 3 und niedriger als oder gleich 6 ist.

8. Verfahren zum Analysieren der Urinprobe gemäß Anspruch 1, wobei das zweite Reagens weiter einen Chelatbildner enthält.

9. Verfahren zum Analysieren der Urinprobe gemäß Anspruch 8, wobei der Chelatbildner Ethylendiamintetraacetat (EDTA-Salz) ist.

10. Verwendung eines Reagens-Kits zur Analyse einer Urinprobe zum Nachweis von Spermien und hefeartigen Pilzen in der Urinprobe durch das Unterscheiden zwischen ihnen, wobei das Reagens-Kits enthält:
ein erstes Reagens, das einen Fluoreszenzfarbstoff enthält, der in der Lage ist, Nukleinsäure zu färben, und
ein zweites Reagens, das Essigsäure und/oder ein Salz davon als ein antiseptisches Mittel und ein Tensid zum Lysieren von Erythrozyten und Beschädigen von Urinpartikeln mit Nukleinsäure, so dass sie für den Fluoreszenzfarbstoff durchlässig sind, enthält.

11. Verwendung des Reagens-Kits zur Analyse einer Urinprobe gemäß Anspruch 10, wobei das Tensid zumindest eines, ausgewählt aus einem kationischen Tensid und einem nicht-ionischen Tensid, ist.

12. Verwendung des Reagens-Kits zur Analyse einer Urinprobe gemäß Anspruch 11, wobei das kationische Tensid ein Tensid vom Typ eines quaternären Ammoniumsalzes und ein Tensid vom Typ eines Pyridiniumsalzes ist; und
wobei das nicht-ionische Tensid ein Polyoxyethylenbasiertes nicht-ionisches Tensid ist.

13. Verwendung des Reagens-Kits zur Analyse einer Urinprobe gemäß Anspruch 11, wobei das kationische Tensid Dodecyltrimethylammoniumbromid ist.

14. Verwendung des Reagens-Kits zur Analyse einer Urinprobe gemäß Anspruch 10, wobei der pH-Wert des zweiten Reagens höher als oder gleich 3 und niedriger als oder gleich 6 ist.

15. Verwendung des Reagens-Kits zur Analyse einer Urinprobe gemäß Anspruch 10, wobei das zweite Reagens weiter einen Chelatbildner enthält.

16. Verwendung des Reagens-Kits zur Analyse einer Urinprobe gemäß Anspruch 15, wobei der Chelatbildner Ethylendiamintetraacetat (EDTA-Salz) ist.

## Revendications

1. Procédé d'analyse d'un échantillon d'urine comprenant les étapes consistant à : préparer un échantillon de mesure en mélangeant l'échantillon d'urine, un premier réactif contenant un colorant fluorescent capable de colorer de l'acide nucléique et, un second réactif contenant de l'acide acétique et/ou un sel de celui-ci en tant qu'agent antiseptique,
obtenir des informations optiques en irradiant des particules urinaires contenues dans l'échantillon de mesure obtenu dans l'étape de préparation avec de la lumière, et
détecter au moins des spermatozoïdes et des organismes analogues à de la levure en tant que particules urinaires sur la base des informations optiques obtenues à l'étape d'obtention.

2. Procédé d'analyse de l'échantillon d'urine selon la revendication 1, dans lequel les informations optiques sont des informations de lumière diffusée et des informations de fluorescence.

3. Procédé d'analyse de l'échantillon d'urine selon la revendication 1, dans lequel le second réactif contient en outre un tensioactif pour lyser des érythrocytes et détériorer des particules urinaires présentant de l'acide nucléique de manière à être perméables au colorant fluorescent.

4. Procédé d'analyse de l'échantillon d'urine selon la revendication 3, dans lequel le tensioactif est au moins un choisi parmi un tensioactif cationique et un tensioactif non ionique.

5. Procédé d'analyse de l'échantillon d'urine selon la revendication 4, dans lequel le tensioactif cationique est choisi parmi un tensioactif de type sel d'ammonium quaternaire et un tensioactif de type sel de pyridinium ; et
dans lequel le tensioactif non ionique est choisi parmi un tensioactif non ionique à base de polyoxyéthylène.

6. Procédé d'analyse de l'échantillon d'urine selon la revendication 4, dans lequel le tensioactif cationique est du bromure de dodécyltriméthylammonium.

7. Procédé d'analyse de l'échantillon d'urine selon la revendication 1, dans lequel le pH du second réactif est supérieur ou égal à 3 et inférieur ou égal à 6.

8. Procédé d'analyse de l'échantillon d'urine selon la revendication 1, dans lequel le second réactif contient en outre un agent chélatant.

9. Procédé d'analyse de l'échantillon d'urine selon la revendication 8, dans lequel l'agent chélatant est l'éthylènediaminetétraacétate (sel d'EDTA).

10. Utilisation d'un kit de réactifs pour l'analyse d'un échantillon d'urine pour détecter des spermatozoïdes et des champignons analogues à de la levure dans l'échantillon d'urine en les différenciant, dans laquelle le kit de réactifs comprend
un premier réactif contenant un colorant fluorescent capable de colorer de l'acide nucléique, et
un second réactif contenant de l'acide acétique et/ou un sel de celui-ci en tant qu'agent antiseptique et un tensioactif pour lyser des érythrocytes et détériorer des particules urinaires présentant un acide nucléique de manière à être perméables au colorant fluorescent.

11. Utilisation du kit de réactifs pour l'analyse d'un échantillon d'urine selon la revendication 10, dans laquelle le tensioactif est au moins un choisi parmi un tensioactif cationique et un tensioactif non ionique.

12. Utilisation du kit de réactifs pour l'analyse d'un échantillon d'urine selon la revendication 11, dans laquelle le tensioactif cationique est un tensioactif de type sel d'ammonium quaternaire et un tensioactif de type sel de pyridinium ; et
dans laquelle le tensioactif non ionique est un tensioactif non ionique à base de polyoxyéthylène.

13. Utilisation du kit de réactifs pour l'analyse d'un échantillon d'urine selon la revendication 11, dans laquelle le tensioactif cationique est du bromure de dodécyltriméthylammonium.

14. Utilisation du kit de réactifs pour l'analyse d'un échantillon d'urine selon la revendication 10, dans laquelle le pH du second réactif est supérieur ou égal à 3 et inférieur ou égal à 6.

15. Utilisation du kit de réactifs pour l'analyse d'un échantillon d'urine selon la revendication 10, dans laquelle le second réactif contient en outre un agent chélatant.

16. Utilisation du kit de réactifs pour l'analyse d'un échantillon d'urine selon la revendication 15, dans laquelle l'agent chélatant est l'éthylènediaminetétraacétate (sel d'EDTA).
